# EUROPÄISCHE PATENTSCHRIFT

(11) **EP 1 087 705 B1**
(45) Veröffentlichungstag und Bekanntmachung des Hinweises auf die Patenterteilung: **03.04.2002**
(21) Anmeldenummer: 99961060.3
(22) Anmeldetag: 03.12.1999
(51) Int. Cl.: A61B 17/24

(54) **MEDIZINISCHES INSTRUMENT FÜR DIE NASENPLASTIK**
MEDICAL INSTRUMENT FOR PERFORMING RHINOPLASTY
INSTRUMENT MEDICAL DESTINE A LA CHIRURGIE PLASTIQUE DU NEZ

(30) Priorität: 16.04.1999 DE 19917287
(43) Veröffentlichungstag der Anmeldung: 04.04.2001
(73) Patentinhaber: Karl Storz GmbH & Co. KG, 78532 Tuttlingen (DE)
(72) Erfinder: Berghaus, Alexander, 10717 Berlin (DE)
(74) Vertreter: Heuckeroth, Volker, Dr.
(86) Internationale Anmeldenummer: EP9909487
(87) Internationale Veröffentlichungsnummer: WO0062686

(56) Entgegenhaltungen:
- WO-A-97/33521
- US-A- 4 457 756
- US-A- 5 308 349
- "Storz - Karl Storz-Endoskope, Band "Endoskope und Instrumente für HNO" 5. Ausgabe" 1996 , KARL STORZ GMBH & CO. , TUTTLINGEN XP002134440 in der Anmeldung erwähnt Seite N15A

## Beschreibung

Die Erfindung betrifft ein medizinisches Instrument für die Nasenplastik, mit einem ersten stabförmigen Element, das einen distalen Abschnitt zum Halten von Nasenknorpel eines Nasenflügels aufweist.

Ein derartiges Instrument ist beispielsweise aus dem DE-Firmenkatalog der Firma Karl Storz GmbH & Co., Tuttlingen "STORZ - Karl Storz-Endoskope", Band "Endoskope und Instrumente für HNO", 5. Ausgabe, 1996, Seite N 15 A, bekannt.

Ein solches Instrument wird auf dem Gebiet der Nasenplastik verwendet. Im Rahmen der Nasenplastik wird die Nasenstruktur unter funktionellen und ästhetischen Gesichtspunkten chirurgisch umgeformt, beispielsweise um die Nase durch Manipulationen am Nasenknorpel zu verkleinern oder um Deformitäten, beispielsweise der Nasenspitze oder der Nasenflügel, zu beseitigen.

Um beispielsweise die Nasenflügelknorpel entsprechend bearbeiten zu können, müssen diese vollständig freigelegt und übersichtlich dargestellt werden, um die teilweise filigranen Manipulationen an ihnen durchführen zu können. Die Nasenflügelknorpel werden dazu zunächst geeignet eingeschnitten, ohne daß jedoch die Flügelknorpel vollständig abgetrennt werden. Die Flügelknorpel müssen dann aus dem Nasenfach der betreffenden Nasenhälfte hervorgezogen und noch von Haut- und Weichgewebe freipräpariert werden. Um nun die gewünschten Manipulationen an dem herausgezogenen Nasenflügelknorpel durchführen zu können, muß dieser in der herausgezogenen Stellung im Bereich vor der Nasenöffnung ruhend gehalten werden.

In dem eingangs genannten DE-Firmenprospekt ist ein mit der Instrumtennummer 478303 bezeichnetes stabförmiges Element in der Art einer Schiene dargestellt, das geeignet ist, den Nasenflügelknorpel einer Nasenhälfte mit seinem distalen Ende zu fassen, aus dem Nasenfach herauszuziehen und dann den Nasenflügelknorpel in der herausgezogenen Stellung zu halten, indem der Nasenflügelknorpel auf dem flächigen distalen Abschnitt des stabförmigen Elements aufgeladen wird. Dazu wird der distale Abschnitt des stabförmigen Elements unter den Nasenflügelknorpel, der noch im Nasenfach gelagert ist, geschoben.

Mit diesem bekannten Instrument kann jedoch nur ein Nasenflügelknorpel in der herausgezogenen Stellung festgehalten werden, so daß die Bearbeitung der Nasenflügelknorpel der linken Nasenhälfte und der rechten Nasenhälfte nacheinander erfolgen muß.

Für den Erfolg der Nasenplastik ist es jedoch entscheidend, daß eine gleichzeitige und symmetrische Bearbeitung beider Nasenflügelknorpel ermöglicht wird, d.h. daß beide Nasenflügelknorpel nebeneinander freigelegt und übersichtlich dargestellt sind, damit dem Chirurg während der Manipulation ständig ein Vergleich zwischen beiden Nasenflügelknorpeln ermöglicht wird.

Um nun beide Nasenflügelknorpel nebeneinander in ihrer herausgezogenen Stellung halten zu können, könnte es in Betracht gezogen werden, zwei der bekannten Instrumente zu verwenden, eines für den Nasenflügelknorpel der linken Nasenhälfte und eines für den Nasenflügelknorpel der rechten Hälfte. Dann besteht jedoch das Problem, daß beide stabförmigen Elemente vom Chirurgen oder einer Assistenz gehalten werden müssen, was den Chirurgen bei der eigentlichen Arbeit, nämlich der Manipulation der Nasenflügelknorpel, behindern würde. Die gleichzeitige Verwendung zweier der bekannten Instrumente erweist sich daher hinsichtlich der Handhabbarkeit eines solchen Instruments als nachteilig.

Der Erfindung liegt daher die Aufgabe zugrunde, ein Instrument der eingangs genannten Art dahingehend weiterzubilden, daß mit dem Instrument eine gleichzeitige Darstellung der Nasenflügelknorpel der linken und der rechten Nasenhälfte ermöglicht wird, wobei das Instrument dabei außerdem leicht zu handhaben ist.

Erfindungsgemäß wird diese Aufgabe hinsichtlich des eingangs genannten Instruments dadurch gelöst, daß das Instrument ein zweites stabförmiges Element, das einen distalen Abschnitt zum Halten von Nasenknorpel des anderen Nasenflügels aufweist, und ein Fixierelement aufweist, das abnehmbar an dem ersten Element und dem zweiten Element angebracht ist, und das die beiden Elemente nebeneinander in etwa paralleler Ausrichtung zueinander aneinander fixiert.

Durch nunmehr zwei vorgesehene stabförmige Elemente können der Nasenflügelknorpel der linken Nasenhälfte und der Nasenflügelknorpel der rechten Hälfte gleichzeitig nebeneinander in herausgezogener Stellung gehalten werden, wodurch beide Nasenflügelknorpel übersichtlich dargestellt werden können und dem Chirurg einen ständigen Vergleich beider Nasenflügelknorpel während der Manipulation an den Knorpeln ermöglichen. Das erfindungsgemäß vorgesehene Fixierelement hat nun den besonderen Vorteil, daß die beiden stabförmigen Elemente, auf denen jeweils ein Nasenflügelknorpel aufgeladen ist, aneinander lagefixiert werden können, so daß die beiden stabförmigen Elemente aufgrund der Fixierung durch das Fixierelement beim üblicherweise während der Operation auf dem Rücken liegenden Patienten bspw. auf dessen Kinn oder einer Beatmungsmaske abgelegt werden können. Es ist daher nicht erforderlich, daß die beiden stabförmigen Elemente von dem Chirurg oder einer Assistenz während der Manipulation an den Nasenflügelknorpeln gehalten werden müssen, da das Fixierelement eine ausreichende Lagefixierung der beiden Elemente gewährleistet. Der Chirurg hat somit beide Hände für die Durchführung der Manipulationen an den Nasenflügelknorpeln frei und ist auch nicht durch eine nahe dabeistehende Assistenz behindert. Dadurch, daß das Fixierelement abnehmbar an dem ersten und dem zweiten Element anbringbar ist, wird der weitere Vorteil erzielt, daß zunächst bei abgenommenem Fixierelement das erste stabförmige Element einzeln bedient werden kann, um den Nasenflügelknorpel beispielsweise der linken Nasenhälfte aufzuladen und herauszuziehen, und daß anschließend das zweite stabförmige Element einzeln bedient werden kann, um den Nasenflügelknorpel der rechten Nasenhälfte aufzuladen und herauszuziehen, was mit einzelnen separaten stabförmigen Elementen einfacher zu bewerkstelligen ist. Nachdem dann beide Nasenflügelknorpel auf das jeweilige stabförmige Element aufgeladen sind, werden die beiden stabförmigen Elemente durch Anbringen des Fixierelements aneinander lagefixiert, und das Instrument kann dann, wie zuvor erwähnt, auf dem Gesicht des Patienten abgelegt werden, und die Nasenflügelknorpel sind zur Manipulation übersichtlich dargestellt. Die Handhabung des erfindungsgemäßen Instruments ist somit besonders einfach. Die Abnehmbarkeit der Fixierelemente hat den weiteren Vorteil, daß die stabförmigen Elemente ohne das Fixierelement auch für andere Zwecke verwendet werden können.

Die der Erfindung zugrunde liegende Aufgabe wird somit vollkommen gelöst.

In einer bevorzugten Ausgestaltung sind der distale Abschnitt des ersten Elements und der distale Abschnitt des zweiten Elements symmetrisch zueinander ausgebildet.

Diese Maßnahme hat den Vorteil, daß im Zusammenhang mit dem Fixierblock eine symmetrische Darstellung der Nasenflügelknorpel ermöglicht wird, wodurch die für den Erfolg der plastischen Operation wünschenswerte symmetrische Behandlung der beiden Flügelknorpel noch erleichtert wird.

In einer weiteren bevorzugten Ausgestaltung weisen der distale Abschnitt des ersten Elements und der distale Abschnitt des zweiten Elements auf ihrer Oberseite Markierungen zur Orientierung auf.

Diese Maßnahme hat den Vorteil, daß die Markierungen, die beispielsweise eine Milimeterskala darstellen, einen genaueren Vergleich der Abmessungen der beiden Nasenflügelknorpel ermöglichen, wodurch eine sehr genaue symmetrische Bearbeitung dieser gewährleistet wird.

In einem bevorzugten Ausführungsbeispiel ist das Fixierelement als vom proximalen Ende der Elemente her aufschiebbares Steckteil ausgebildet.

Hierdurch ergibt sich ein vorteilhafterweise besonders einfach zu handhabender Verbindungsmechanismus zwischen dem Fixierelement und den beiden stabförmigen Elementen, wenn die beiden stabförmigen Elemente nach dem Aufladen der beiden Nasenknorpel einander fixiert werden sollen. Das Aufschieben des Fixierelements vom proximalen Ende der Elemente her hat den Vorteil, daß keine Kräfte quer zur Längsrichtung der Elemente zum Anbringen des Fixierelements aufgebracht werden müssen, so daß vermieden wird, daß die stabförmigen Elemente durch Verschwenken aus ihrer Längsrichtung unerwünschte Hebelkräfte auf die aufgeladenen Nasenknorpel ausüben können.

Dabei ist es weiterhin bevorzugt, wenn das Fixierelement zwei sich längs erstreckende Öffnungen zur Aufnahme von proximalen Längsabschnitten der Elemente aufweist.

Hierbei ist von Vorteil, daß das Fixierelement die beiden stabförmigen Elemente an deren proximalen Ende über sich längs erstreckende Abschnitte desselben umgreift, wodurch eine besonders stabile Lagefixierung der beiden Elemente nebeneinander erreicht wird, bei der ein Verkippen der beiden Elemente aus ihrer zueinander etwa parallelen Ausrichtung vermieden wird.

Hierbei ist weiterhin bevorzugt, wenn das Fixierelement ein Blockteil aufweist, in dem die Öffnungen als Nuten eingebracht sind, wobei das Fixierelement weiterhin einen Deckel aufweist, der die Nuten an der offenen Längsseite verschließt.

Diese zweiteilige Ausgestaltung des Fixierelements ist fertigungstechnisch besonders vorteilhaft, da die Nuten durch einen Fräsvorgang ins Blockteil eingebracht werden können. Dadurch können Öffnungen in dem Fixierelement auch mit nicht rundem Querschnitt eingebracht werden, um eine formschlüssige Aufnahme der beiden Elemente im Fall, daß deren proximalen Abschnitte ebenfalls einen nicht runden Querschnitt aufweisen, zu ermöglichen. Der Deckel verschließt dann vorteilhaft die Nuten an deren offenen Längsseite, so daß die Öffnungen seitlich allseitig geschlossen sind und eine sichere Fixierung des Fixierelements an den beiden Elementen ermöglicht wird. Der Deckel kann mit dem Blockteil fest verbunden, beispielsweise verschweißt sein.

Bevorzugt ist es jedoch auch, wenn der Deckel lösbar an dem Blockteil befestigt ist.

Hierbei ist von Vorteil, daß sich in den Öffnungen ansammelnder Schmutz nach Lösen des Deckels leichter entfernen läßt, wodurch die Reinigbarkeit des Fixierelements verbessert wird. Außerdem hat dies den Vorteil, daß sich die Elemente im Fall, daß sie sich in dem Fixierelement verkanten und somit nicht mehr leicht aus dem Fixierelement herausziehen lassen, nach Abnehmen des Deckels leichter entfernen lassen. Der Deckel kann beispielsweise mittels Schrauben oder durch Verrasten an dem Blockteil lösbar befestigt sein.

In einer weiteren bevorzugten Ausgestaltung ist das Fixierelement als seitlich auf die Elemente aufsteck- oder aufschiebbares Steckteil ausgebildet.

Auch dies stellt eine vorteilhafte Möglichkeit eines Verbindungsmechanismus zum Anbringen des Fixierelements an den beiden Elementen dar.

In einer weiteren bevorzugten Ausgestaltung ist das Fixierelement selbsthemmend an den Elementen befestigbar.

Hierbei ist von Vorteil, daß das Fixierelement nicht durch zusätzliche Maßnahmen, wie Schrauben oder dergleichen, an den beiden stabförmigen Elementen befestigt werden muß. Bei dem zuvor erwähnten Ausführungsbeispiel, bei dem das Fixierelement als vom proximalen Ende her aufschiebbares Steckteil ausgebildet ist, kann dies beispielsweise durch ein elastisches Element, beispielsweise in Form einer gebogenen Blattfeder realisiert sein, die die proximalen Längsabschnitte der beiden stabförmigen Elemente in den Öffnungen durch Klemmwirkung arretiert.

Dabei ist es auch bevorzugt, wenn das Fixierelement an den beiden Elementen verrastbar ist.

Auch dies führt zu einer vorteilhaft einfach zu handhabenden Fixierung des Fixierelements an den beiden stabförmigen Elementen, beispielsweise, indem an dem Fixierelement in den Öffnungen eine elastische Rastnase vorgesehen ist, die in eine Rastkerbe an den in den Öffnungen aufgenommenen Längsabschnitten der beiden stabförmigen Elemente beim Aufschieben des Fixierelements einrastet.

Weitere Vorteile ergeben sich aus der nachfolgenden Beschreibung und der beigefügten Zeichnung.

Es versteht sich, daß die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird hiernach mit Bezug auf diese näher beschrieben. Es zeigen:
- Fig. 1: ein erfindungsgemäßes Instrument im zusammengesetzten Zustand in einer perspektivischen Ansicht;
- Fig. 2: ein stabförmiges Element des Instruments in Fig. 1 in Alleinstellung in Seitenansicht;
- Fig. 3: das Element in Fig. 2 in Draufsicht;
- Fig. 4: ein Fixierelement des Instruments in Fig. 1 in einem horizontalen Längsschnitt;
- Fig. 5: das Fixierelement in Fig. 4 in einem Schnitt entlang der Linie V-V in Fig. 4;
- Fig. 6: einen der Fig. 4 entsprechenden Schnitt durch ein Blockteil des Fixierelements;
- Fig. 7: einen Schnitt durch das Blockteil in Fig. 6 entlang der Linie VII-VII in Fig. 6;
- Fig. 8: eine Draufsicht auf das Blockteil in Fig. 6; und
- Fig. 9: eine schematische Darstellung der Funktion des Instruments bei der Verwendung in der Nasenplastik.

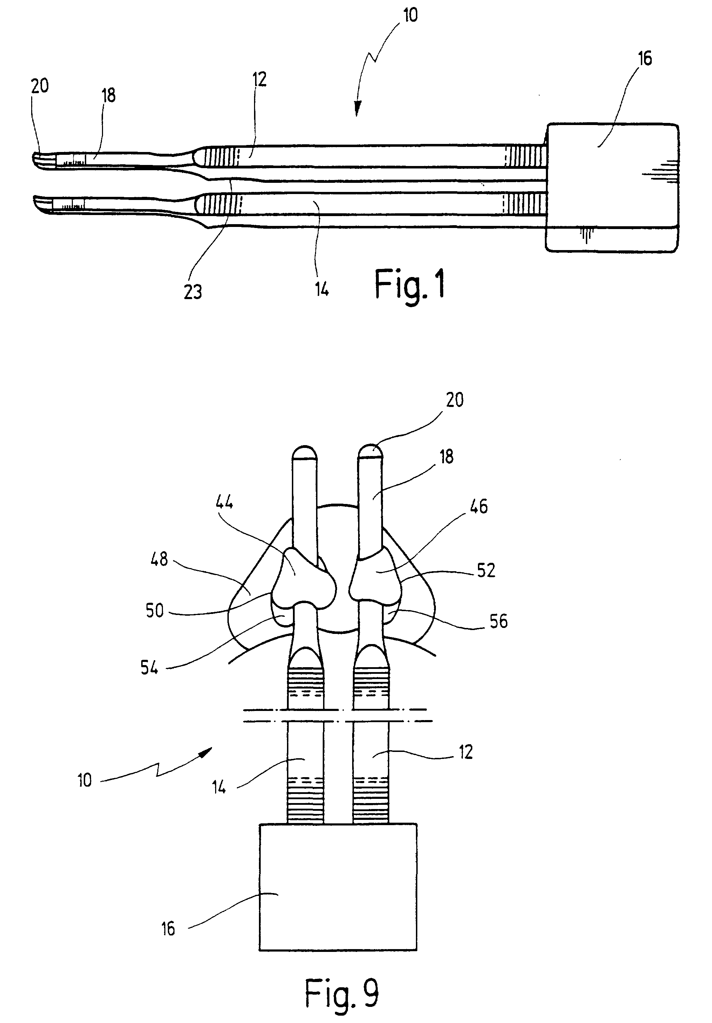

In Fig. 1 ist ein mit dem allgemeinen Bezugszeichen 10 versehenes medizinisches Instrument dargestellt, das in der Nasenplastik bei der chirurgischen Umformung der Nasenstruktur einer Nase verwendet wird, wie später in Bezug auf Fig. 9 noch beschrieben wird.

Das Instrument 10 weist ein erstes stabförmiges Element 12 sowie ein zweites stabförmiges Element 14 auf.

Das erste stabförmige Element 12 und das zweite stabförmige Element 14 sind mittels eines Fixierelementes 16 nebeneinander in etwa paralleler Ausrichtung aneinander fixiert. Das Fixierelement 16 ist dabei abnehmbar an den Elementen 12 und 14 angebracht. Die Elemente 12 und 14 sowie das Fixierelement 16 sind aus einem für medizinische Zwecke geeigneten Stahl gefertigt.

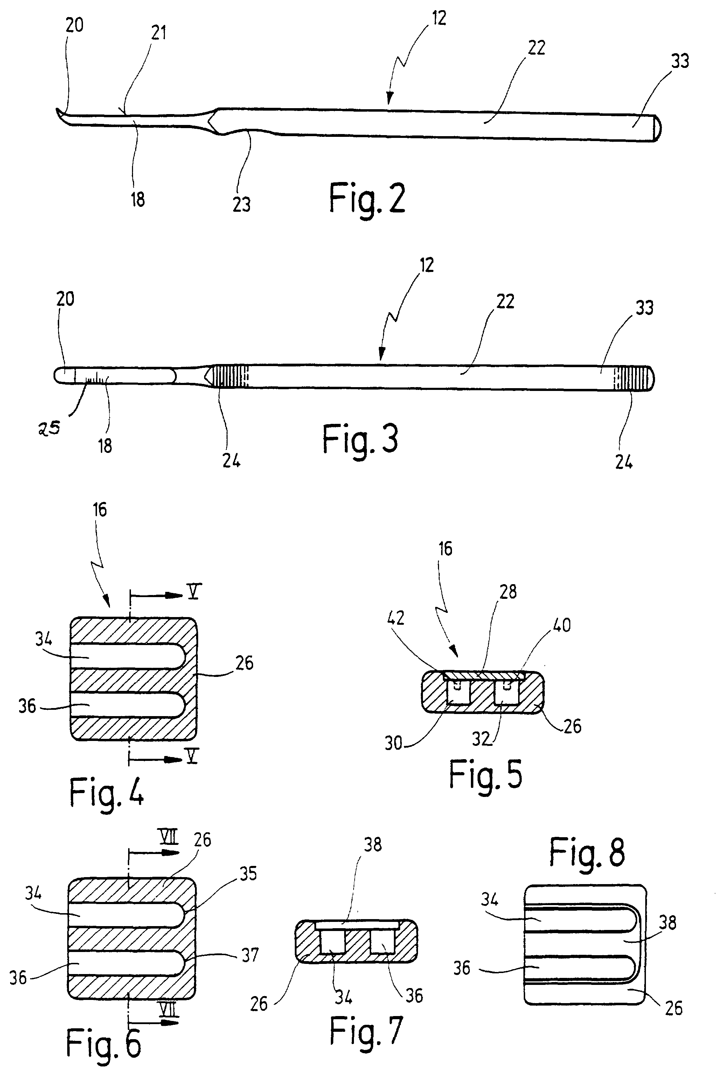

Mit Bezug auf Figuren 2 und 3 wird nun die Ausgestaltung des ersten stabförmigen Elements 12 näher beschrieben, wobei die Ausgestaltung des zweiten stabförmigen Elements 14 mit der Ausgestaltung des ersten stabförmigen Elements 12 identisch ist.

Das stabförmige Element 12 weist einen distalen Abschnitt 18 auf, dessen äußere distale Spitze 20 geringfügig aus der Längsachse des stabförmigen Elements 12 heraus gekrümmt ist. Der distale Abschnitt 18 des stabförmigen Elements 12 dient dazu, den Nasenflügelknorpel einer Nasenhälfte zu halten, indem der Nasenflügelknorpel auf eine obere Seite 21, die flächig eben ausgebildet ist, aufgeladen wird, wobei die zur oberen Seite 21 hin gekrümmte Spitze 20 ein Abrutschen des Nasenflügelknorpels vermeidet. Der distale Abschnitt 18 des stabförmigen Elements 12 ist im Querschnitt halbkreisförmig aus Vollmaterial ausgebildet, d.h. eine der oberen Seite 21 gegenüberliegende untere Seite ist rund ausgebildet.

Auf seiner oberen Seite 21 weist der distale Abschnitt 18 eine Markierung 23 zur Orientierung auf, die zusammen mit einer entsprechenden Markierung auf dem distalen Abschnitt des Elements 14 als Vergleichsmaßstab bei der Bearbeitung der beiden Nasenknorpel dient. Eine solche Markierung 23 kann in Form einer Millimeterskala mit entsprechenden Linien auf der oberen Seite 21 vorgesehen sein.

An den distalen Abschnitt 18 des stabförmigen Elements 12 schließt sich ein Elementkörper 22 an, der ebenfalls aus einem Vollmaterial gebildet und im Querschnitt rechteckig ausgebildet ist.

Auf einer Unterseite des Elementkörpers 22 ist nahe bei dem distalen Abschnitt 18 eine muldenartige Vertiefung 23 ausgebildet, wodurch eine ergonomische Handhabung des Elements 12 ermöglicht wird.

Auf der Unterseite und der dieser gegenüberliegenden oberen Seite weist der Elementkörper 22 axial durchgehend eine Riffelung 24 auf, die die Griffigkeit des stabförmigen Elements 12 bei dessen Handhabung erhöht. Die Riffelung 24 ist in Fig. 3 aus Gründen der Vereinfachung der Darstellung nicht durchgehend gezeigt.

Die stabförmigen Elemente 12 und 14 sind in der Art von Schienen oder meiselähnlichen Instrumenten ausgebildet, wobei die distale Spitze 20 des distalen Abschnitts 18 auch eine geschärfte distale Kante aufweisen kann, um beispielsweise schabend oder schneidend zu wirken.

Weiterhin sind der distale Abschnitt 18 des Elements 12 und der entsprechende Abschnitt des Elements 14 symmetrisch zueinander ausgebildet.

In Figuren 4 bis 8 ist das Fixierelement 16 im einzelnen dargestellt.

Das Fixierelement 16 ist insgesamt zweiteilig ausgebildet. Das Fixierelement 16 weist ein Blockteil 26 sowie einen Deckel 28 auf.

In dem Fixierelement 16 ist eine erste Öffnung 30 und eine zweite Öffnung 32 ausgebildet, die jeweils im Querschnitt rechteckig ausgebildet sind und deren Querschnittsflächen geringfügig größer sind als die Querschnittsfläche des Elementkörpers 22 des stabförmigen Elements 12 bzw. des entsprechenden Elementkörpers des zweiten stabförmigen Elements 14, so daß proximale Längsabschnitte 33 des stabförmigen Elements 12 bzw. 14 formschlüssig in den Öffnungen 30 und 32 aufgenommen werden, wenn das Fixierelement 16 an den Elementen 12 und 14 angebracht ist.

Die Öffnungen 30 und 32 sind als Nuten 34 und 36 in dem Blockteil 26 des Fixierelements 16 ausgebildet, die sich nahezu über die gesamte Länge des Blockteils 26 erstrecken, wobei ein proximales Ende 35 der Nut 34 und ein proximales Ende 37 der Nut 36 geschlossen ist. Die Nuten 34 und 36 werden bei der Herstellung des Blockteils 26 in dieses eingefräst.

Der Deckel 28 verschließt die Nuten 34 und 36 an ihrer offenen Seite, so daß die Öffnungen 30 und 32 seitlich allseitig geschlossen sind, und nur an ihrem distalen Ende offen sind. Der Deckel 28 ist in einer Vertiefung 38 in dem Blockteil 26 formschlüssig eingepaßt, so daß das Fixierelement eine glatte, stufenfreie Oberfläche besitzt und insgesamt die Form eines flachen Quaders aufweist.

Der Deckel 28 ist mit dem Blockteil 26 durch Schweißen, Kleben, oder dergleichen, fest verbunden.

Es kann jedoch auch vorgesehen sein, den Deckel 28 lösbar mit dem Blockteil 26 zu verbinden, beispielsweise durch Schrauben oder durch einen Rastmechanismus, so daß der Deckel 28 bei Bedarf von dem Blockteil 26, beispielsweise zum Reinigen des Fixierelements 16, abgenommen werden kann.

Das Fixierelement 16 ist mit der zuvor beschriebenen Ausgestaltung als vom proximalen Ende her auf das stabförmige Element 12 bzw. das stabförmige Element 14 aufschiebbares Steckteil ausgebildet.

Bei einer anderen nicht dargestellten Ausgestaltung kann das Fixierelement jedoch auch in Form eines von der Seite her auf die stabförmigen Elemente 12 und 14 aufsteck- oder aufclipsbares Steckteil ausgebildet sein. Beispielsweise kann auch das Fixierelement 16 ohne den Deckel 28, d.h. das Blockteil 26 mit den seitlich offenen Nuten 34 und 36 als seitlich aufclipsbares Fixierelement verwendet werden, wozu noch entsprechende Rastmittel in den Nuten 34 und 36 vorgesehen werden können, um das Blockteil 26 dann auch ohne den Deckel 28 sicher an den stabförmigen Elementen 12 und 14 zu fixieren.

Das Fixierelement 16 ist weiterhin selbsthemmend an den stabförmigen Elementen 12 und 14 befestigbar. In Fig. 5 ist dazu schematisch mit unterbrochenen Linien ein elastisches Element 40 in der Öffnung 32 sowie ein elastisches Element 42 in der Öffnung 30 dargestellt, die beispielsweise als Blattfedern ausgebildet sind, die an dem Deckel 28 befestigt sind, und die die proximalen Längsabschnitte 33 der stabförmigen Elemente 12 und 14 in den Öffnungen 30 und 32 einklemmen.

Es können auch nicht dargestellte Rastmittel an dem Fixierelement 16 in den Öffnungen 30 und 32 vorgesehen sein, beispielsweise Rastnasen, die mit entsprechenden Rastkerben in den proximalen Längsabschnitten oder auch mit der Riffelung 24 derselben verrastend zusammenwirken.

Im folgenden wird nun mit Bezug auf Fig. 9 die Funktion des Instruments 10 bei der Verwendung in der Nasenplastik zum Darstellen der Nasenflügelknorpel einer Nase näher beschrieben.

In Fig. 9 ist eine Nase mit dem Bezugszeichen 48, ein Nasenflügelknorpel der rechten Nasenhälfte mit dem Bezugszeichen 44 und ein Nasenflügelknorpel der linken Nasenhälfte mit dem Bezugszeichen 46 versehen.

Um die Nasenflügelknorpel 44 und 46, wie in Fig. 9 dargestellt, darstellen zu können, werden die Nasenflügelknorpel 44 und 46 vorher mittels anderer geeigneter Instrumente durch Einbringen von Teilschnitten teilweise gelöst, wobei die Nasenflügelknorpel 44 und 46 nicht vollständig abgetrennt werden, sondern in ihrem jeweiligen Nasenfach noch mit dem übrigen Knorpel an einer Verbindungsstelle 50 bzw. 52 verbunden bleiben. Die Schnitte werden dabei so gelegt, daß sich die Nasenflügelknorpel 44 und 46 aus den Nasenlöchern 54 und 56 herausziehen bzw. herausklappen lassen.

Zum Herausziehen und damit Darstellen der Nasenflügelknorpel 44 und 46 wird nun das Instrument 10 verwendet.

Bei zunächst abgenommenem Fixierelement 16 wird zunächst mittels einem der stabförmigen Elemente, beispielsweise dem stabförmigen Element 12 der linke Nasenflügelknorpel 46 in seinem Nasenfach in der Nasenöffnung 56 gefaßt, indem der distale Abschnitt 18 mit seiner Spitze 20 unter den teilweise abgeschnittenen Nasenflügelknorpel 46 geschoben wird und aus der Nasenöffnung 56 herausgezogen wird. Ist der Nasennflügelknorpel 46 aus der Nasenöffnung 52 herausgezogen, wird der distale Abschnitt 18 vor der Nasenöffnung 56, wie in Fig. 9 dargestellt, positioniert, so daß ein Zurückklappen des Nasenflügelknorpels 46 in das linke Nasenfach verhindert wird. Der Nasenflügelknorpel 46 ist somit dargestellt.

Im anschließenden Schritt wird unter Verwendung des zweiten stabförmigen Elements 14 mit dem rechten Nasenflügelknorpel 44 ebenso verfahren, bis dieser ebenfalls aus dem Nasenfach herausgezogen und übersichtlich dargestellt ist.

Anschließend wird nun das Fixierelement 16 auf die stabförmigen Elemente 12 und 14 von deren proximalen Ende her aufgeschoben, wodurch die Elemente 12 und 14 in paralleler Ausrichtung zueinander und etwa dem Abstand der Nasenlöcher 54 und 56 entsprechend voneinander beabstandet aneinander lagefixiert sind.

Die das Instrument 10 bildende Anordnung aus dem stabförmigen Element 12, dem stabförmigen Element 14 und dem Fixierelement 16 bildet nun eine starre Einheit, die auf dem Gesicht des Patienten abgelegt werden kann, wobei das Fixierelement 16 etwa auf dem Kinn des Patienten zu liegen kommt. Im folgenden kann nun der Chirurg die erforderlichen Manipulationen, d.h. Veränderungen an den Nasenflügelknorpeln 44 und 46 vornehmen, ohne daß er oder ein Assistent das Instrument 10 halten muß. Das Instrument 10 ermöglicht somit eine symmetrische Darstellung und Behandlung der beiden Nasenflügelknorpel 44 und 46 nebeneinander.

## Patentansprüche

1. Medizinisches Instrument für die Nasenplastik, mit einem ersten stabförmigen Element (12), das einen distalen Abschnitt (18) zum Halten von Nasenknorpel eines Nasenflügels aufweist, **gekennzeichnet durch** ein zweites stabförmiges Element (14), das einen distalen Abschnitt zum Halten von Nasenknorpel des anderen Nasenflügels aufweist, und **durch** ein Fixierelement (16), das abnehmbar an dem ersten Element (12) und dem zweiten Element (14) angebracht ist, und das die beiden Elemente (12, 14) nebeneinander in etwa paralleler Ausrichtung zueinander aneinander fixiert.

2. Instrument nach Anspruch 1, **dadurch gekennzeichnet, daß** der distale Abschnitt (18) des ersten Elements (12) und der distale Abschnitt des zweiten Elements (14) symmetrisch zueinander ausgebildet sind.

3. Instrument nach Anspruch 1 oder 2, **dadurch gekennzeichnet, daß** der distale Abschnitt (18) des ersten Elements (12) und der distale Abschnitt des zweiten Elements (14) auf ihrer Oberseite Markierungen zur Orientierung aufweisen.

4. Instrument nach einem der Ansprüche 1 bis 3, **dadurch gekennzeichnet, daß** das Fixierelement (16) als vom proximalen Ende der Elemente (12, 14) her aufschiebbares Steckteil ausgebildet ist.

5. Instrument nach Anspruch 4, **dadurch gekennzeichnet, daß** das Fixierelement (16) zwei sich längs erstreckende Öffnungen (30, 32) zur Aufnahme von proximalen Längsabschnitten (33) der Elemente (12, 14) aufweist.

6. Instrument nach Anspruch 5, **dadurch gekennzeichnet, daß** das Fixierelement (16) ein Blockteil (26) aufweist, in dem die Öffnungen (30, 32) als Nuten (34, 36) eingebracht sind, wobei das Fixierelement (16) weiterhin einen Deckel (28) aufweist, der die Nuten (34, 36) an deren offenen Längsseite verchließt.

7. Instrument nach Anspruch 6, **dadurch gekennzeichnet, daß** der Deckel (28) lösbar an dem Blockteil (26) befestigt ist.

8. Instrument nach einem der Ansprüche 1 bis 3, **dadurch gekennzeichnet, daß** das Fixierelement (16) als seitlich auf die Elemente (12, 14) aufsteck- oder aufclipsbares Steckteil ausgebildet ist.

9. Instrument nach einem der Ansprüche 1 bis 8, **dadurch gekennzeichnet, daß** das Fixierelement (16) selbsthemmend an den Elementen (12, 14) befestigbar ist.

10. Instrument nach Anspruch 9, **dadurch gekennzeichnet, daß** das Fixierelement (16) an den beiden Elementen (12, 14) verrastbar ist.

## Claims

1. Medical instrument for rhinoplasty, comprising a first rod-shaped element (12) having a distal portion (18) for holding nasal cartilage of a nose side, **characterized by** a second rod-shaped element (14) having a distal portion for holding nasal cartilage of the other nose side, and by a fixing element (16), which is detachably mounted on said first element (12) and second element (14), and which fixes the two elements (12, 14) one beside the other in approximately parallel alignment one relative to the other.

2. The instrument of claim 1, **characterized in that** the distal portion (18) of the first element (12) and the distal portion of the second element (14) are configured symmetrically one relative to the other.

3. The instrument of claim 1 or 2, **characterized in that** the distal portion (18) of the first element (12) and the distal portion of the second element (14) comprise markings for orientation on their upper sides.

4. The instrument of anyone of claims 1 through 3, **characterized in that** the fixing element (16) is configured as a slip-on element slidable from the proximal end of the elements (12, 14).

5. The instrument of claim 4, **characterized in that** the fixing element (16) comprises two longitudinally extending openings (30, 32) for receiving proximal longitudinal portions (33) of the elements (12, 14).

6. The instrument of claim 5, **characterized in that** the fixing element (16) comprises a block member (26) in which the openings (30, 32) are provided as grooves (34, 36), wherein the fixing element (16) further comprises a cover (28) that closes the grooves (34, 36) at their open long side.

7. The instrument of claim 6, **characterized in that** the cover (28) is detachably fixed at the block member (26).

8. The instrument of anyone of claims 1 through 3, **characterized in that** the fixing element (16) is configured as a slip-on element that can be slipped or clipped onto the elements (12, 14) from the side.

9. The instrument of anyone of claims 1 through 8, **characterized in that** the fixing element (16) can be fixed on the elements (12, 14) in a self-locking fashion.

10. The instrument of claim 9, **characterized in that** the fixing element (16) can be snap-locked on the two elements (12, 14).

## Revendications

1. Instrument médical pour la chirurgie plastique du nez, avec un premier élément (12) en forme de barrette qui comporte une portion distale (18) destinée à maintenir le cartilage d'une aile du nez, **caractérisé par** un deuxième élément (14) en forme de barrette qui comporte une portion distale destinée à maintenir le cartilage de l'autre aile du nez, et par un élément de fixation (16) qui est fixé de manière amovible au premier élément (12) et au deuxième élément (14), et qui fixe côte à côte les deux éléments (12, 14) dans une orientation approximativement parallèle entre eux.

2. Instrument selon la revendication 1, **caractérisé en ce que** la portion distale (18) du premier élément (12) et la portion distale du deuxième élément (14) sont conformées de manière symétrique l'une de l'autre.

3. Instrument selon la revendication 1 ou 2, **caractérisé en ce que** la portion distale (18) du premier élément (12) et la portion distale du deuxième élément (14) présentent des marquages d'orientation sur leur face supérieure.

4. Instrument selon l'une des revendications 1 à 3, **caractérisé en ce que** le l'élément de fixation (16) est réalisé en tant qu'élément à emboîter, qui peut être emboîté à partir de l'extrémité proximale des éléments (12, 14).

5. Instrument selon la revendication 4, **caractérisé en ce que** l'élément de fixation (16) comporte deux ouvertures (30, 32) s'étendant longitudinalement, pour recevoir des portions longitudinales proximales (33) des éléments (12, 14).

6. Instrument selon la revendication 5, **caractérisé en ce que** l'élément de fixation (16) comporte un bloc (26) dans lequel les ouvertures (30, 32) sont pratiquées sous forme de rainures (34, 36), l'élément de fixation (16) comportant en outre un couvercle (28) qui ferme les rainures (34, 36) sur leur côté longitudinal ouvert.

7. Instrument selon la revendication 6, **caractérisé en ce que** le couvercle (28) est fixé de manière non permanente au bloc (26).

8. Instrument selon l'une des revendications 1 à 3, **caractérisé en ce que** l'élément de fixation (16) est réalisé en tant qu'élément à emboîter, qui peut être emboîté ou clipsé latéralement sur les éléments (12, 14).

9. Instrument selon l'une des revendications 1 à 8, **caractérisé en ce que** l'élément de fixation (16) peut être fixé de manière autobloquante aux éléments (12, 14).

10. Instrument selon la revendication 9, **caractérisé en ce que** l'élément de fixation (16) peut s'accrocher aux deux éléments (12, 14).
